# EUROPÄISCHE PATENTANMELDUNG

(11) **EP 2 394 629 A2**
(43) Veröffentlichungstag der Anmeldung: **14.12.2011**
(21) Anmeldenummer: 11167652.4
(22) Anmeldetag: 26.05.2011
(51) Int. Cl.: A61K 8/11, A61K 8/41, A61K 8/73, A61Q 5/06

(54) **Kosmetische Zusammensetzung zum Färben der Haare mit einem direktziehenden Haarfarbstoff und mit einem Trägersystem aus Polysacchariden**

(30) Priorität: 11.06.2010 DE 102010030000
(71) Anmelder: ROVI Cosmetics International GmbH, 36381 Schlüchtern (DE)
(72) Erfinder: Beyer, Monika, 61130 Nidderau-Ostheim (DE); Teichmüller, Dirk, 63589 Linsengericht (DE)
(74) Vertreter: WSL Patentanwälte

(57) **Zusammenfassung**

Die vorliegende Erfindung betrifft kosmetische Zusammensetzungen zum Färben der Haare mit wenigstens einem direktziehenden Haarfarbstoff und mit einem Trägersystem für den wenigstens einen direktziehenden Haarfarbstoff sowie die Verwendung solcher Zusammensetzungen in kosmetischen Formulierungen zum Färben der Haare. Um eine Möglichkeit bereitzustellen, mit der die Dauerhaftigkeit der Bindung von direktziehenden Haarfarbstoffen an das Haar verbessert werden kann mit dem Ziel, dass direktziehende Farbstoffe möglichst lang am Haar verbleiben, um die gewünschte Haarfarbe möglichst lang in der gewünschten Qualität zu liefern, werden erfindungsgemäß Zusammensetzungen der vorgenannten Art vorgeschlagen, bei denen das Trägersystem vesikulär ist und aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen.

## Beschreibung

Die vorliegende Erfindung betrifft eine kosmetische Zusammensetzung zum Färben der Haare mit wenigstens einem direktziehenden Haarfarbstoff, der in einem vesikulären Trägersystem verkapselt ist, sowie die Verwendung einer solchen Zusammensetzung in entsprechenden kosmetischen Formulierungen zum Färben der Haare.

Viele Menschen wünschen sich vorübergehend oder dauerhaft eine andere als ihre natürliche Haarfarbe, und es gibt eine ganze Reihe von Verfahren, um seinem Haar eine andere Farbe zu verleihen.

Bei einem dieser Verfahren werden direktziehende Haarfarbstoffe verwendet, die sich an der Cuticula des Haares anlagern oder sich darin einlagern. Bei diesem semi-permanenten oder temporären Verfahren handelt sich damit um eine Färbung, bei der das natürlich vorhandene Pigment des Haares nicht verändert wird, so dass sich zusammen mit der natürlichen Haarfarbe der neue Farbton ergibt. Im Gegensatz dazu gibt es auch Verfahren mit Haarfarbstoffen, die die natürliche Haarfarbe chemisch verändern, indem Sie bspw. chemisch an bestimmte Aminosäuren im Haar binden.

Viele direktziehende Haarfarbstoffe lagern sich von Hause aus nicht mit der gewünschten Dauerhaftigkeit an die Cuticula an bzw. in die Cuticula ein und manche dringen - beispielsweise wegen Ihrer Molekülgröße - überhaupt nicht bis in den Cortex des Haares vor. Haarfärbemittel mit solchen Haarfarbstoffen führen daher oft nicht zu einer Färbung mit der gewünschten Dauerhaftigkeit. Es besteht daher ein Bedarf nach einer Möglichkeit, die Dauerhaftigkeit der An- bzw. Einlagerung von direktziehenden Haarfarbstoffen zu verlängern.

Der vorliegenden Erfindung liegt daher die Aufgabe zugrunde, eine Möglichkeit bereitzustellen, mit der die Dauerhaftigkeit der Bindung von direktziehenden Haarfarbstoffen an bzw. in das Haar verbessert werden kann. Ziel ist es, dass erreicht wird, dass direktziehende Farbstoffe möglichst lang am Haar verbleiben, um die gewünschte Haarfarbe möglichst lang in der gewünschten Qualität zu liefern.

Diese Aufgabe wird erfindungsgemäß gelöst durch eine kosmetische Zusammensetzung zum Färben der Haare mit wenigstens einem direktziehenden Haarfarbstoff, der in einem vesikulären Trägersystem verkapselt ist, wobei das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen.

Direktziehende Farbstoffe liefern vermittelt durch das Trägersystem der erfindungsgemäßen Zusammensetzung die gewünschte Haarfarbe länger in der gewünschten Qualität als ohne das Trägersystem der erfindungsgemäßen Zusammensetzung, was wohl darauf zurückzuführen sein wird, dass direktziehende Farbstoffe vermittelt durch das Trägersystem der erfindungsgemäßen Zusammensetzung länger auf dem Haar verbleiben.

Vermutlich wird durch das Trägersystem der erfindungsgemäßen Zusammensetzung erreicht, dass die Dauerhaftigkeit der Anlagerung von direktziehenden Haarfarbstoffen an das Haar verbessert wird, indem der Farbstoff fester auf dem Haar anhaftet um dort angelagert zu werden, wobei diese Feststellungen in Bezug auf die vorliegende Erfindung jedoch nicht bindend sein sollen, und der Umfang der Erfindung durch diese Feststellungen auch nicht beschränkt werden soll.

Zusätzlich bewirkt die erfindungsgemäße Zusammensetzung eine bessere Stabilisierung des Haarfarbstoffs, was zu einer Erhöhung der Menge an intaktem Haarfarbstoff, die an den Haaren bereitgestellt wird, führt, was wiederum den Effekt hat, dass die gewünschte Haarfarbe länger in der gewünschten Qualität aufrechterhalten werden kann.

Der Begriff "Verkapselung" umfasst im Zusammenhang mit der vorliegenden Erfindung die prinzipielle Einbettung von direktziehenden Haarfarbstoffen zwischen den Polysaccharidmolekülen und den von den Polysaccharidmolekülen ausgebildeten Nanostrukturen sowie den Einschluss der direktziehenden Haarfarbstoffe im Vesikelinneren.

Die erfindungsgemäßen Vesikel haben ein Zetapotential im Bereich von 1 bis 150 mV. Der Begriff "Zetapotential" beschreibt das elektrische Potential einer Abscherschicht eines bewegten Partikels in einer Suspension. Die Messung des Zetapotentials kann dadurch erfolgen, dass man Partikel durch ein angelegtes elektrisches Feld bewegt. Aus der resultierenden Geschwindigkeit der Partikel lässt sich dann das Zetapotential berechnen. Bei manchen Ausführungsformen weisen die Vesikel ein Zetapotential von 30 bis 100 mV auf. Bei bestimmten Lipidvesikeln beträgt das Zetapotential 40 bis 60 mV.

Das Zetapotential wird im Zusammenhang mit der vorliegenden Erfindung mittels Laser-Doppler-Elektrophorese bestimmt. Dabei erfolgen die Messungen in stark verdünnten wässrigen Kochsalzlösungen, wobei die zu vermessenden Proben üblicherweise in Konzentrationen von 0,01-0,1 Gew.-% in 1 mM Natriumchloridlösung vorliegen. Die pH-Werte der Probenlösungen liegen im pH-Wert-Spezifikationsbereich der jeweiligen zu vermessenden Produkte (erfindungsgemäße UV-Schutzmittel und Formulierungen), wobei im Regelfall produktspezifisch pH-Werte im Bereich von pH 5,5 bis maximal pH 7,5 getroffen werden.

Die verbesserte Adhäsion der Zusammensetzung basiert u.a. auf der positiven Ladung der Vesikeloberfläche. Die positive Ladung der Vesikeloberfläche führt zu einer verbesserten Anhaftung der Vesikel an der Oberfläche von Haaren. Es hat sich außerdem gezeigt, dass diese verbesserte Adhäsion auch mit der erfindungsgemäß nano-skaligen Teilchengröße der Trägervesikel zusammenhängt, wobei all diese Feststellungen in Bezug auf die vorliegende Erfindung jedoch nicht bindend sein sollen und den Umfang der Erfindung daher auch nicht beschränken sollen.

Die Teilchengröße der erfindungsgemäßenvesikel ist in dem Bereich von 10 bis 1000 nm. Bei bestimmten Ausführungsformen ist die Teilchengröße 100 bis 400 nm. Bei anderen Ausführungsformen ist sie 100 bis 350 nm oder in dem Bereich 100 bis 250 nm.

Der Begriff "Teilchengröße" bedeutet im Zusammenhang mit der vorliegenden Erfindung die mittlere Teilchengröße. Die mittlere Teilchengröße ist mittels Photonenkorrelationsspektroskopie bestimmbar. Die Photonenkorrelationsspektroskopie - auch als dynamische Lichtstreuung bezeichnet - ist ein optisches Messverfahren zur Bestimmung der Größenverteilung von Vesikeln und Partikeln in Flüssigkeiten. Die Methode nutzt die Streuung von Laserlicht durch die Vesikel aus.

Durch die erfindungsgemäße Kombination von positiver Ladung der Vesikeloberfläche und nanoskaliger Teilchengröße der Trägervesikel wird erreicht, dass in den Vesikeln enthaltene direktziehende Haarfarbstoffe dauerhafter auf bzw. in den Haaren verbleiben.

Vorzugsweise wird die positive Ladung der Vesikeloberfläche dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber ausgewählt sind unter primären, sekundären, tertiären und quarternären Alkyl-Ammoniumsalzen oder Kombinationen davon. Besonders bevorzugt sind hierbei C₁₂-C₂₂- Alkyl-Ammoniumsalze.

Ganz besonders bevorzugt sind als Ladungsgeber bei der vorliegenden Erfindung quarternäre C₁₂-C₂₂-Alkyl-Trimethylammoniumsalze (bzw. Fettsäure-Trimoniumsalze) der Formel 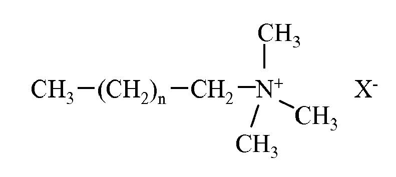 wobei n eine ganze Zahl von 10 bis 22 und X⁻ ein anorganisches oder organisches Anion ist. Vorzugsweise ist n in der oben angegebenen Alkyl-Trimethylammoniumsalzformel gleich 22.

Vorzugsweise ist X⁻ in der oben angegebenen Formel ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, Iodid, Saccharinat, Tosylat oder Methosulfat.

Vorzugsweise sind die Ladungsgeber bei der vorliegenden Erfindung ausgewählt unter einem oder mehreren von Ceteartrimoniumchlorid, Cetrimoniumbromid, Cetrimoniumchlorid, Cetrimoniumsaccharinat, Cetrimoniumtosylat, Cocotrimoniumchlorid, Cocotrimoniummethosulfat, Lautrimoniumbromid, Lautrimoniumchlorid, Myrtrimoniumbromid, Octyldecyltrimoniumchlorid, Steartrimoniumbromid, Steartrimoniumchlorid, Steartrimoniummethosulfat und Steartrimoniumsaccharinat. Bei einer besonders bevorzugten Ausführungsform der vorliegenden Erfindung wird Behentrimoniumchlorid als Ladungsgeber verwendet.

Vorzugweise werden bei der vorliegenden Erfindung einer oder mehrere der vorgenannten positiv geladenen Ladungsgeber so eingesetzt, dass der Ladungsgeberanteil in den Vesikeln bezogen auf das Gesamtgewicht der Vesikel insgesamt in dem Bereich von 0,01 bis 10 Gew.-% liegt. Bei bestimmten Ausführungsformen liegt der Ladungsgeberanteil in dem Bereich von 0,01 bis 2,0 Gew.-%.

Die in Gewichtsprozenten angegebenen Anteile beziehen sich prinzipiell auf die vollständige Formulierung des Haarfärbemittels gemäß Rezepturvorgaben, inklusive des zur Vesikelbildung erforderlichen Wasseranteils, wenn nicht etwas anderes angegeben ist.

Der angegebene Anteil umfasst außerdem alle eindeutig unter die Definition der jeweiligen Substanzgruppe fallenden Substanzen. Demnach umfasst der angegebene Ladungsgeberanteil bei den Ausführungsformen mit einem Ladungsgeber den Anteil dieses einen Ladungsgebers und bei Ausführungsformen mit mehreren Ladungsgebern die Summe aller Ladungsgeber. Die gilt im Übrigen in gleicher Weise für im Zusammenhang mit dieser Erfindung angegebene Anteile an hydrophobiertem Polysaccharid, Filtersubstanz und Hilfsstoff oder anderen Substanz- bzw. Stoffanteilen.

Das Polysaccharidgrundgerüst der hydrophobierten Polysaccharide, die bei dem Haarfärbemittel für die Bildung der Vesikel verwendet werden können, kann unter allen kosmetisch oder pharmazeutisch verträglichen Polysacchariden ausgewählt werden, die in der Lage sind, Vesikel zu bilden. Vorzugsweise handelt es sich um wasserlösliche Polysaccharide und/oder um deren Ether mit kurzkettigen Alkoholen (C₁ bis C₄), wobei die wasserlöslichen Polysaccharide linear, verzweigt, kammartig und/oder sternförmig sein können. Besonders bevorzugt sind lineare wasserlösliche Polysaccharide. Es kommen auch Copolymere oder Block-Copolymere unterschiedlicher Monosaccharid-Einheiten und/oder auf unterschiedliche Weise miteinander verknüpfter Monosaccharid-Einheiten in Betracht.

Die hydrophobierten Polysaccharide, aus denen die Vesikel aufgebaut sind, weisen vorzugsweise ein Polysaccharid-Grundgerüst aus Polyglucose oder Polyfructose auf. Bevorzugte hydrophobierte Polysaccharide mit einem Polysaccharid-Grundgerüst aus einer Polyglucose sind Cellulose, Methylcellulose, Hydroxyethylcellulose, Amylose, Amylopektin und Dextrin. Ein bevorzugtes hydrophobiertes Polysaccharid mit einem Polysaccharid-Grundgerüst aus Polyfructose ist Inulin.

Das hydrophobierte Polysaccharid umfasst bei der vorliegenden Erfindung vorzugsweise im Mittel von 5 bis 1000 Monosaccharid-Einheiten. Noch bevorzugter sind von 10 bis 500 Monosaccharid-Einheiten. Bei bestimmten Ausführungsformen sind von 20 bis 100 Monosaccharid-Einheiten bevorzugt.

Bei speziellen Ausführungsformen können auch Gemische der oben genannten Polysaccharide eingesetzt werden mit der Maßgabe, dass diese Gemische in der Lage sind, Vesikel zu bilden.

Der Anteil der Polysaccharide in den Vesikeln liegt bezogen auf das Gesamtgewicht der Vesikel in dem Bereich von 1 bis 85 Gew.-%. Vorzugsweise beträgt der Polysaccharidanteil bezogen auf das Gesamtgewicht der Vesikel 5 bis 25 Gew.-%. Bei bestimmten Ausführungsformen beträgt der Polysaccharidanteil 8 bis 15 Gew.-%.

Bei bevorzugten Ausführungsformen sind die hydrophobierten Polysaccharide, aus denen die Vesikel aufgebaut sind, dadurch hydrophobiert, dass sie ein Polysaccharid-Grundgerüst mit C₃-₂₂-Alkylgruppen, die über Alkyl-Etherbindungen oder über Alkyl-Urethanbindungen an die Hydroxygruppen des Polysaccharids gebunden sind, oder die über einen Linker (z.B. Polyether-Linker, Polyethylenglykol-Linker) an das Polysaccharid-Grundgerüst gebunden sind, aufweisen.

Das Molekulargewicht des hydrophob modifizierten Polysaccharids liegt bei bevorzugten Ausführungsformen in dem Bereich von 5000 bis 500.000 g/mol. Bevorzugt ist hierbei der Bereich von 5000 bis 100.000 g/mol.

Bei bevorzugten hydrophob modifizierten Polysacchariden beträgt der Quotient aus Anzahl an hydrophob modifizierenden Gruppen und modifizierbaren Gruppen (Modifizierungsgrad) von 0,01 bis 0,9. Bei besonders bevorzugten Ausführungsformen beträgt der Modifizierungsgrad von 0,03 bis 0,15.

Die hydrophoben Gruppen und auch das Polysaccharid-Rückgrat können bei bestimmten Ausführungsformen durch beispielsweise Halogen-, Hydroxy-, Alkoxy-, Amino-, Alkylamino-, Aryl-, Arylalkyl-, Carboxy-, Carboxyester- und cycloaliphatische Reste ein- oder mehrfach substituiert sein. Bevorzugt handelt es sich bei den hydrophob modifizierten Polysacchariden jedoch um nichtionische Verbindungen.

Als direktziehende Haarfarbstoffe kommen bei der vorliegenden Erfindung uneingeschränkt alle laut Kosmetikverordnung gegenwärtig zugelassenen und künftig zugelassenen wasser- und fettlöslichen direktziehenden Haarfarbstoffe sowie beliebige Kombinationen dieser Farbstoffe in Betracht, die verkapselt werden können. Hierbei kann es sich sowohl um lipophile (öllösliche) als auch um hydrophile (wasserlösliche) Farbstoffe handeln, die aus natürlichen Quellen isoliert oder chemisch oder biotechnologisch hergestellt wurden, wie z.B. Pflanzenfarbstoffe wie Henna oder Indigo, Inoaniline, Indophenole, Nitrobenzolderviate, Anthrachinon-Farbstoffe, Triphenylmethanfarbstoffe, Chinonfarbstoffe, Azofarbstoffe, kationische und anionische Farbstoffe.

Ein Haarfarbstoff ist im Sinne der vorliegenden Erfindung dann lipophil, wenn er bei 25°C einen n-Oktanol-Wasser-Verteilungskoeffizient K_{ow} aufweist, der größer als 1 ist. Ein Haarfarbstoff ist im Sinne der vorliegenden Erfindung dann hydrophil, wenn er bei 25°C einen n-Oktanol-Wasser-Verteilungskoeffizient K_{ow} aufweist, der kleiner oder gleich 1 ist.

Beispiele für direktziehende Haarfarbstoffe, die bei der vorliegenden Erfindung zur Anwendung kommen können, sind ohne Beschränkung hierauf:

**Tabelle 1: direktziehende Haarbfarbstoffe**

| **Colipa No.** | **INCI** | **CAS-Nr.** | **EINECS-Nr.** | **Color-Index-Nr** |
|---|---|---|---|---|
| B001 | Acid Yellow 1 | | | |
| B005 | Disperse Red 17 | 3179-89-3 | 221-665-5 | CI 11210 |
| B007 | Basic Brown 17 | 68391-32-2 | 269-944-0 | CI 12251 |
| B024 | 4-NITRO-o-PHENYLENEDIAMINE | 99-56-9 | 202-766-3 | CI 76020 |
| B031 | HC RED NO. 13 | 29705-39-3 | 94158-13-1 | |
| B034 | N,N'-BIS(2-HYDROXYETHYL)-2-NITRO-p-PHENYLENEDIAMINE | 84041-77-0 | 281-856-4 | |
| B036 | HC RED NO. 7 | 24905-87-1 | 246-521-9 | |
| B037 | HC BLUE NO. 2 | 33229-34-4 | 251-410-3 | |
| B047 | HC ORANGE NO. 1 | 54381-08-7 | 259-132-4 | |
| B048 | HC RED NO. 1 | 2784-89-6 | 220-494-3 | |
| B051 | 4-AMINO-3-NITROPHENOL | 610-81-1 | 210-236-8 | |
| B052 | 2-HYDROXYETHYLAMINO-5-NITROANISOLE | 66095-81-6 | 266-138-0 | |
| B054 | 3-NITRO-p-HYDROXYETHYLAMINOPHENOL | 65235-31-6 | 265-648-0 | |
| B055 | 2-AMINO-3-NITROPHENOL | 603-85-0 | | |
| B056 | 6-NITRO-o-TOLUIDINE | 570-24-1 | 209-329-3 | |
| B058 | 3-METHYLAMINO-4-NITROPHENOXY-ETHANOL | 59820-63-2 | 261-940-7 | |
| B060 | 2-NITRO-5-GLYCERYL METHYLANILINE | 80062-31-3 | 279-383-3 | |
| B063 | HC YELLOW NO. 11 | 73388-54-2 | - | |
| B066 | HC VIOLET NO. 1 | 82576-75-8 | 417-600-7 | |
| B067 | HC ORANGE NO. 2 | 85765-48-6 | 416-410-1 | |
| B069 | HC YELLOW NO. 9 | 141973-33-3 | 86419-69-4 | |
| B070 | 4-NITROPHENYL AMINOETHYLUREA | 27080-42-8 | 410-700-1 | |
| B071 | HC RED NO. 10 AND HC RED NO. 11 | 95576-89-9 + 95576-92-4 | 408-240-1 | |
| B072 | 2-HYDROXYETHYL PICRAMIC ACID | 99610-72-7 | 412-520-9 | |

| **Colipa No.** | **INCI** | **CAS-Nr.** | **EINECS-Nr.** | **Color-Index-Nr.** |
|---|---|---|---|---|
| B073 | HC BLUE NO. 12 | 132885-85-9 | 407-020-2 | |
| B075 | HYDROXYETHYL-2-NITRO-P-TOLUIDINE | 100418-33-5 | 408-090-7 (ELINCS) | |
| B077 | HC BLUE NO. 11 | 23920-15-2 | 459-980-7 | |
| B080 | HC YELLOW NO. 7 | 104226-21-3 | | |
| B087 | 4-AMINO-2-NITROPHENYL-AMINE-2'-CARBOXYLIC ACID | 117907-43-4 | - | |
| B089 | 2-CHLORO-6-ETHYLAMINO-4-NITROPHENOL | 131657-78-8 | 411-440-1 | |
| B098 | HC VIOLET NO. 2 | | | |
| B099 | 2-Amino-6-Chloro-4-Nitrophenol | 6358-09-4 | 228-762-1 | |
| B100 | 4-Hydroxypropylamino-3-Nitrophenol | 92952-81-3 | 406-305-9 | |
| B102 | HC YELLOW NO. 13 | 10442-83-8 | | |
| B104 | 1,2,3,4-TETRAHYDRO-6-NITROCHINOXALIN | 158006-54-3 (hydrochloride) | - | |
| B113 | Basic Orange No.69 | 226940-14-3 | - | CI 112605 |
| B115 | Basic Violet 2 | 3248-91-7 | 221-831-7 | CI 42520 |
| C008 | Basic Red 76 | 68391-30-0 | 269-941-4 | CI 12245 |
| C009 | Basic Brown 16 | 26381-41-9 | 247-640-9 | CI 12250 |
| C010 | Basic Yellow 57 | 68391-31-1 | 269-943-5 | CI 12719 |
| C015 | Orange 4 | 633-96-5 | 211-199-0 | CI 15510 |
| C022 | Red 33 | 3567-66-6 | 222-656-9 | CI 17200 |
| C029 | Yellow 5 - Acid Yellow 23 | 1934-21-0 | 217-699-5 | CI 19140 |
| C040 | Acid Blue 9 - Blue 1 | 3844-45-9 | 4223-333-98 | CI 42090 |
| C046 | Basic Blue 7 | 2390-60-5 | | CI 42595 |
| C053 | Acid Red 92 | 18472-87-2 | 242-355-6 | CI 45410 |
| C054 | Yellow 10 - Acid Yellow 3 | - | - | CI 47005 |
| C059 | Basic Blue 99 | 68123-13-7 | - | CI 56059 |
| C063 | Acid Violet 43 - Ext. Violet 2 | 4430-18-6 | 224-618-7 | CI 60730 |
| C064 | Disperse Violet 1 | 128-95-0+116-85-8 | - | |
| C067 | ACID BLUE 62 | 4368-56-3 | 224-460-9 | CI 62045 |
| C117 | HYDROXYANTHRAQUINONEAMINOPROPYLMETHYL MORPHOLINIUM METHOSULFATE | 38866-20-5 | 254-161-9 | |
| C119 | HC RED NO. 8 | 13556-29-1 | 306-778-0 | |
| C129 | HC GREEN NO. 1 | 52136-25-1 | 257-687-7 | |
| C146 | LAWSONE | 83-72-7 | - | CI 75480 |
| C169 | Henna (Lawsonia inermis) | 84988-66-9 | - | |
| C172 | HC BLUE No. 14 | 99788-75-7 | - | |
| C174 | CURRY RED | 25956-17-6 | 247-368-0 | CI 16035 |
| C175 | Acid Red 18 | 2611-82-7 | 220-036-2 | CI 16255 |
| C177 | Acid Red 52 | 3520-42-1 | 222-529-8 | CI 45100 |
| C178 | Acid Green 25 | 4403-90-1 | 224-546-6 | CI 61570 |

Vorzugsweise beträgt der Anteil an direktziehendem Haarfarbstoff bei der vorliegenden Erfindung bezogen auf die gesamte Zusammensetzung 0,01 bis 40 Gew.-%.

Bei bestimmten Ausführungsformen liegen Kombinationen aus zwei oder mehreren direktziehenden Haarfarbstoffen vor, wobei die Summe dieser Farbstoffe bezogen auf die gesamte Zusammensetzung 0,01 bis 40 Gew.-% ausmacht.

Bei bestimmten Ausführungsformen weist die erfindungsgemäße Zusammensetzung zusätzlich wenigstens eine UV-Filtersubstanz mit einer chemischen oder physikalischen UV-Filterwirkung auf.

Als UV-Filtersubstanz kommen hierbei uneingeschränkt alle chemisch oder physikalisch UVfilternd wirkenden Substanzen in Betracht, vorausgesetzt, sie sind in Öl löslich. Die chemischen Filtersubstanzen absorbieren energiereiche Strahlung und geben sie als energieärmere, langwelligere Strahlung oder Wärme wieder ab. Die physikalischen Filtersubstanzen streuen und reflektieren das Licht hauptsächlich.

Bevorzugte Beispiele für UV-Filtersubstanzen, die bei der vorliegenden Erfindung zur Anwendung kommen können, sind ohne Beschränkung hierauf 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kalium-phenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

Vorzugsweise beträgt der Anteil an UV-Filtersubstanz bei der vorliegenden Erfindung 1 bis 65 Gew.-% bezogen auf das Gesamtgewicht der vollständigen Formulierung des Haarfärbemittels gemäß Rezepturvorgaben, inklusive des zur Vesikelbildung erforderlichen Wasseranteils.

Da die einzelnen Filtersubstanzen in der Regel keinen Schutz über das gesamte UV-Spektrum hinweg bieten, werden bei bevorzugten Ausführungsformen der Erfindung mehrere Filtersubstanzen kombiniert, um einen möglichst breiten UV-Schutz zu erreichen.

Die erfindungsgemäße Zusammensetzung kann außerdem alle Hilfs- und Zusatzstoffe, die üblicherweise bei kosmetischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Vesikel und deren Stabilität einwirken und/oder der Konservierung der Haarfärbemittel dienen. Beispiele für solche Hilfsstoffe sind Öle, Alkohole, Polyole, Antioxidantien, Gelbildner, Puffer, Konservierungsmittel, Bakterizide und Keimhemmer, Konsistenzgeber, Verdicker und Komplexbildner.

Vorzugsweise ist die Zusammensetzung gemäß der vorliegenden Erfindung dadurch gekennzeichnet, dass der Hilfsstoffanteil bezogen auf das Gesamtgewicht der vollständigen Formulierung des Haarfärbemittels gemäß Rezepturvorgaben, inklusive des zur Vesikelbildung erforderlichen Wasseranteils in dem Bereich von 0,01 bis 10 Gew.-% liegt.

Erfindungsgemäß wird die Zusammensetzung zum Färben der Haare vorzugsweise in kosmetischen Formulierungen zum Färben der Haare verwendet. Die erfindungsgemäße Zusammensetzung kann dabei in allen zum Färben der Haare geeigneten Formulierungen vorliegen, beispielsweise in Form eines farbgebenden Shampoos oder farbgebenden Schaumfestigers oder in Form von Tönungslotionen, Schaumtönungen oder als Tönungsmousse.

Der Anteil der Farbstoffe an der Gesamtformulierung beträgt vorzugsweise 0,001 - 10 %.

Selbstverständlich handelt es sich bei allen Komponenten der erfindungsgemäßen Zusammensetzung und von dessen Formulierungen um kosmetisch verträgliche Stoffe. Ein Stoff ist im Sinne dieser Erfindung kosmetisch verträglich, wenn er nicht toxisch ist und bei der Mehrzahl der potentiellen Anwender anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

## Patentansprüche

1. Kosmetische Zusammensetzung zum Färben der Haare mit wenigstens einem direktziehenden Haarfarbstoff, der in einem vesikulären Trägersystem verkapselt ist, **dadurch gekennzeichnet, dass** das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen.

2. Zusammensetzung gemäß Anspruch 1, **dadurch gekennzeichnet, dass** der Haarfarbstoffanteil in den Vesikeln bezogen auf das Gesamtgewicht der Zusammensetzung in dem Bereich von 1 bis 65 Gew.-% liegt.

3. Zusammensetzung gemäß Anspruch 2, **dadurch gekennzeichnet, dass** der Polysaccharidanteil in den Vesikeln bezogen auf das Gesamtgewicht der Vesikel in dem Bereich von 1 bis 85 Gew.-% liegt.

4. Zusammensetzung gemäß einem der vorangehenden Ansprüche, **dadurch gekennzeichnet, dass** die positive Oberflächenladung dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber ausgewählt sind unter primären, sekundären, tertiären und quarternären Alkyl-Ammoniumsalzen oder Kombinationen davon.

5. Zusammensetzung gemäß Anspruch 4, **dadurch gekennzeichnet, dass** der Ladungsgeberanteil in den Vesikeln bezogen auf das Gesamtgewicht der Zusammensetzung in dem Bereich von 0,01 bis 10 Gew.-% liegt.

6. Zusammensetzung gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aus Polyglucose oder Polyfructose aufweisen.

7. Zusammensetzung gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aus einer Polyglucose, die unter Cellulose, Methylcellulose, Hydroxyethylcellulose, Amylose, Amylopektin oder Dextrin ausgewählt ist, oder ein Polysaccharid-Grundgerüst aus der Polyfructose Inulin aufweisen.

8. Zusammensetzung gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aufweisen, das durch C₃-₂₂-Alkylgruppen, die über Alkyl-Etherbindungen oder über Alkyl-Urethanbindungen an die Hydroxygruppen des Polysaccharids gebunden sind, oder die über einen Linker an das Polysaccharid-Grundgerüst gebunden sind, hydrophob modifiziert sind.

9. Zusammensetzunggemäß einem der vorangehenden Ansprüche, **dadurch gekennzeichnet, dass** sie zusätzlich wenigstens eine UV-Filtersubstanz mit einer chemischen oder physikalischen UV-Filterwirkung aufweist.

10. Zusammensetzung gemäß Anspruch 9, **dadurch gekennzeichnet, dass** die wenigstens eine UV-Filtersubstanz ausgewählt ist unter 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kaliumphenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

11. Zusammensetzung gemäß einem der Ansprüche 1 bis 10, **dadurch gekennzeichnet, dass** es zusätzlich einen oder mehrere Hilfsstoffe umfasst.

12. Zusammensetzung gemäß einem der Ansprüche 1 bis 11, **dadurch gekennzeichnet, dass** der Hilfsstoffanteil in den Vesikeln bezogen auf das Gesamtgewicht der Zusammensetzung in dem Bereich von 0,01 bis 10 Gew.-% liegt.

13. Verwendung einer Zusammensetzung gemäß einem der Ansprüche 1 bis 12 zur Herstellung einer kosmetischen Formulierung zum Färben der Haare.

14. Kosmetische Formulierung, die eine Zusammensetzung gemäß einem der Ansprüche 1 bis 12 enthält.

15. Formulierung nach Anspruch 13 **dadurch gekennzeichnet, dass** der Farbstoffanteil in der Formulierung bezogen auf deren Gesamtgewicht in dem Bereich von 0,001 - 10 Gew.-% liegt.
